# EUROPEAN PATENT SPECIFICATION

(11) **EP 2 492 356 B1**
(45) Date of publication and mention of the grant of the patent: **21.08.2013**
(21) Application number: 12157307.5
(22) Date of filing: 28.02.2012
(51) Int. Cl.: C12Q 1/68

(54) **Method of detecting methylated DNA in sample**
Verfahren zum Nachweis von methyliertem DNA in einer Probe
Procédé de détection de l'ADN méthylé dans un échantillon

(30) Priority: 28.02.2011 JP 2011042367
(43) Date of publication of application: 29.08.2012
(73) Proprietor: SYSMEX CORPORATION, Hyogo 651-0073 (JP)
(72) Inventor: Sakai, Ayako, Kobe-shi, Hyogo 651-0073 (JP); Kajita, Masahiro, Kobe-shi, Hyogo 651-0073 (JP)
(74) Representative: Paemen, Liesbet R.J.

(56) References cited:
- US-A1- 2010 105 058
- SMIRNIKHINA S A ET AL: "Common methods for cytosine methylation analysis in DNA", MOLECULAR BIOLOGY (MOSCOW), vol. 43, no. 3, June 2009 (2009-06), pages 355-359, XP002675793,
- ACEVEDO LUIS G ET AL: "Novel DNA binding domain-based assays for detection of methylated and nonmethylated DNA", EPIGENOMICS, vol. 3, no. 1, 1 February 2011 (2011-02-01), pages 93-101, XP002675794,
- FRAGA M F ET AL: "DNA Methylation: A Profile of Methods and Applications", BIOTECHNIQUES, INFORMA HEALTHCARE, US, vol. 33, no. 3, 1 September 2002 (2002-09-01), pages 632,634-636, XP003015596, ISSN: 0736-6205

## Description

### FIELD OF THE INVENTION

The present invention relates to a method of detecting methylated DNA in a sample containing DNA.

### BACKGROUND

In chromosomal DNA of higher eukaryotes, it is known that the 5th position of cytosine (C) among bases forming DNA is methylated. Such DNA methylation serves as a control mechanism of gene expression. For example, when a CpG-rich region (it is also called "CpG island" or "CG island") present in a promoter region of a certain gene is methylated, transcription of the gene is suppressed. This phenomenon is also called "gene silencing". On the other hand, when the CpG island is not methylated, a transcription factor can be bound to the promoter. This allows for the transcription of the gene.

As described above, DNA methylation is one of control mechanisms of gene expression and plays an important role in various physiological and pathological phenomena such as early embryonic development, tissue-specific gene expression, genomic imprinting and X chromosome inactivation which are phenomena unique to mammals, chromosome stabilization, and timing of DNA replication. Furthermore, it has been revealed that abnormalities in methylation of DNA, i.e., gene silencing due to the DNA methylation, is involved in diseases such as cancer. Thus, the importance of detecting methylated DNA in various genes has been recently increased.

As for the method of detecting methylated DNA, various methods are known in the art. Examples thereof include a bisulfite sequencing method, a method of using a methylation-sensitive restriction enzyme, and a method of using methylated DNA immunoprecipitation (the MeDIP method). In the bisulfite sequencing method, unmethylated cytosine in DNA is converted to uracil by an effect of bisulfite, the base sequence is determined, and a methylated site in DNA is detected. In the method of using a methylation-sensitive restriction enzyme, the fact that the methylation-sensitive restriction enzyme cannot cleave the methylated recognition sequence is utilized and methylated DNA is detected by examining the cleavage results. In the MeDIP method, immunoprecipitation is performed using an antibody specifically recognizing methylated DNA or a methylated DNA binding protein, the obtained methylated DNA is subjected to microarray analysis, and the methylated DNA is detected. This method is known as the MeDIP-Chip method.

Japanese Patent Application Laid-Open (JP-A) No. 2008-263961 discloses a method of detecting methylated DNA using a methylation-sensitive restriction enzyme comprising: immobilizing methylated DNA in a sample on a solid phase using an anti-methylated DNA antibody; and preforming digestive treatment with a methylation-sensitive restriction enzyme.

Thus, since the importance of detection of methylated DNA is increasing, there is a demand for development of a novel method of detecting methylated DNA.

### SUMMARY OF THE INVENTION

The scope of the present invention is defined solely by the appended claims, and is not affected to any degree by the statements within this summary.

An objective of the present invention is to provide a novel method of detecting methylated DNA in a sample.

The present inventors have surprisingly found that when a protein capable of binding to methylated DNA is bound to methylated DNA, the methylated DNA is not completely degradated by deoxyribonuclease and still remains, and the obtained deoxyribonuclease degradation product does not have an affect in a detection process. Thus, the present invention has been completed.

(1) A first aspect of the present invention is a method of detecting methylated DNA in a sample, comprising:
   bringing a sample which may include methylated DNA into contact with a protein capable of binding to methylated DNA to bind the methylated DNA in the sample to the protein;
   bringing the sample obtained in the binding process into contact with at least one deoxyribonuclease to degrade DNA in the sample; and
   detecting methylated DNA which is not degraded by the deoxyribonuclease by the binding of the sample obtained by the degradation process to the protein ; wherein
   the at least one deoxyribonuclease is a deoxyribonuclease different from a methylation-sensitive restriction enzyme capable of degradating single-stranded DNA.
(2) The method according to (1), wherein
   a protein capable of binding to the methylated DNA is an anti-methylated DNA antibody or a methylated DNA binding protein.
(3) The method according to (1), wherein
   the protein capable of binding to the methylated DNA is an anti-methylated DNA antibody.
(4) The method according to (1), wherein
   the protein capable of binding to the methylated DNA is at least one methylated DNA binding protein selected from the group consisting of MBD1, MBD2, MBD4, and MeCP2.
(5) The method according to any one of (1) to (4), wherein
   the sample is cultured cells or a sample prepared from blood, body fluids, tissues or cells collected from organisms.
(6) The method according to any one of (1) to (5), wherein
   the deoxyribonuclease is at least one selected from the group consisting of deoxyribonuclease I, exonuclease I, lambda exonuclease, T7 exonuclease, exonuclease III, RecJ exonuclease, exonuclease T, BAL31 nuclease, Mung Bean nuclease, the Micrococcaceae nuclease, and T7 endonuclease.
(7) The method according to any one of (1) to (6), wherein
   the sample is a sample which may include single-stranded methylated DNA and
   single-stranded methylated DNA is detected in the process of detecting methylated DNA.
(8) The method according to any one of (1) to (7), wherein
   methylated DNA is detected using a nucleic acid amplification method, a nucleotide sequencing method or a microarray method in the process of detecting methylated DNA.

The present invention provides a novel method of detecting methylated DNA in a sample. According to the present invention, it is possible to omit cleaning and purifying processes which are needed to improve detection accuracy in conventional methods of detecting methylated DNA. In this case, methylated DNA in a sample can be more simply detected.

### BRIEF DESCRIPTION OF THE DRAWINGS

Fig. 1 is an electrophoresis photograph obtained by subjecting a solution of 6MeCG oligonucleotide (i.e., a synthetic oligonucleotide) as a sample to a detection method of the present invention using DNaseI as deoxyribonuclease and detecting the oligonucleotide by PCR assay;
Fig. 2 is an electrophoresis photograph obtained by subjecting a solution of 6MeCG oligonucleotide (i.e., a synthetic oligonucleotide) as a sample to the detection method of the present invention using Exonuclease I as deoxyribonuclease and detecting the oligonucleotide by PCR assay;
Fig. 3 is an electrophoresis photograph obtained by subjecting genomic DNA obtained from breast cancer cell line MCF7 as a sample to the detection method of the present invention using DNaseI as deoxyribonuclease and detecting methylated DNA by PCR assay; and
Fig. 4 is an electrophoresis photograph obtained by subjecting genomic DNA obtained from MCF7 cells as a sample to the detection method of the present invention using Exonuclease I as deoxyribonuclease and detecting methylated DNA by PCR assay.

### DETAILED DESCRIPTION OF THE PREFERRED EMBODIMENTS

The preferred embodiments of the present invention will be described hereinafter with reference to the drawings.

The term "CpG site" used herein means a site where a cytosine (C) is adjacent to a guanine (G) in the base sequence of DNA in this order in a direction from 5' to 3'. The character of "p" of CpG represents a phosphodiester linkage between cytosine and guanine.
The term "methylated CpG site" means a CpG site in which the 5th position of cytosine is modified by methylation. That is, at the methylated CpG site, 5-methylcytosine (methylated cytosine) is adjacent to guanine in this order in a direction from 5' to 3'.
The term "methylated DNA" used herein means DNA containing at least one 5-methylcytosine.

In the method of detecting methylated DNA in a sample of the present invention (hereinafter also called a "detection method"), a sample which may include methylated DNA is brought into contact with the protein capable of binding to methylated DNA. When methylated DNA is present in the sample, the methylated DNA can be bound to the above protein because of the contact (hereinafter, the process is called a "binding process").
In the detection method of the present invention, the methylated DNA bound to the above protein is not completely degradated by deoxyribonuclease which is used in the following process. That is, the binding to the above protein allows 5-methylcytosine in methylated DNA and its peripheral region to be saved from degradation by deoxyribonuclease.

The sample to be subjected to the detection method of the present invention is not particularly limited as long as it is the sample which may include methylated DNA. Examples of the sample include a DNA containing sample prepared from a biological sample and a sample containing a synthetic polynucleotide which contains at least one 5-methylcytosine. Examples of the biological sample include cultured cells or blood, body fluids, tissues and cells collected from organisms. The "blood" used herein may be any of whole blood and serum and blood plasma obtained from the whole blood. The terms "tissues" and "cells" used herein include cultures of the tissues and cells collected from organisms.

When the sample which may include methylated DNA is a sample prepared from blood, body fluids, tissues or cells collected from organisms, the results of the detection method of the present invention can be used for the diagnosis and determination of diseases associated with methylated DNA.

In the embodiments of the present invention, preparation of a DNA containing sample from a biological sample can be performed by a known method in the art. For example, a sample can be prepared by the steps including mixing a solubilized liquid which contains a surfactant for solubilizing cells or tissues (sodium cholate, sodium dodecyl sulfate, etc.) with a biological sample, subjecting the mixture to physical processes (stirring, homogenization, ultrasonic fragmentation, etc.), releasing DNA included in the biological sample into a solubilized liquid, and extracting the DNA. In the embodiments of the present invention, the extracted DNA may be purified by a known method in the art. The extraction and purification of DNA from the biological sample can be performed using a commercially available kit.

In the embodiment of the present invention, DNA in a sample is preferably a DNA fragment with 100 to 1000 bp. The methylated DNA can be efficiently bound to the protein capable of binding to methylated DNA by fragmenting the DNA included in the sample to the length.
DNA fragmentation can be performed by a known method in the art, such as a physical process, a chemical process or a restriction enzyme process. As the physical process, for example, ultrasonic fragmentation is cited. As the chemical process, for example, alkaline treatment using sodium hydroxide is cited. In the restriction enzyme process, the restriction enzyme can be appropriately selected based on a base sequence of a target DNA. For example, MseI, BamHI, and the like can be used. In the embodiments of the present invention, it is preferable that DNA in a sample is fragmented by the physical process.

In the embodiments of the present invention, methylated DNA which may be included in a sample may be single-stranded methylated DNA. That is, the above sample is a sample which may include single-stranded methylated DNA. The case where a process of detecting methylated DNA to be described later is a process of detecting single-stranded methylated DNA is included in the scope of the present invention.
In the embodiments of the present invention, a process of modifying the DNA included in the sample to a single-strand may be performed before the binding process. The process is known in the art. For example, the DNA in the sample can be modified to a single-strand by heating the sample which may include methylated DNA at about 95°C and immediately cooling it to 4°C.

In the embodiments of the present invention, the protein capable of binding to methylated DNA is not particularly limited as long as it is a protein capable of recognizing and binding to 5-methylcytosine in DNA or the methylated CpG site. Examples of the protein include an anti-methylated DNA antibody and a methylated DNA binding protein. Among them, the anti-methylated DNA antibody is preferred. Particularly, when single-stranded methylated DNA is detected, it is desirable to use the anti-methylated DNA antibody.

Examples of the anti-methylated DNA antibody include an anti-methylated cytosine antibody and an anti-methylated CpG antibody. In the embodiments of the present invention, an anti-methylated cytidine antibody can be used as the anti-methylated DNA antibody.
In the embodiments of the present invention, the anti-methylated DNA antibody may be either a polyclonal antibody or a monoclonal antibody. As the anti-methylated DNA antibody, an active fragment which is obtained by fragmenting the anti-methylated DNA antibody may be used. The active fragment is not particularly limited as long as it is a fragment which does not lose a specific binding activity to methylated DNA. Examples thereof include a Fab fragment, a F(ab')₂ fragment, and a sFv fragment. The active fragment can be prepared, for example, by cleaving a purified anti-methylated DNA monoclonal antibody by an enzyme such as papain, pepsin or trypsin.

In the embodiments of the present invention, a commercially available anti-methylated DNA antibody may be used. Examples of the commercially available anti-methylated DNA antibody include antibodies shown in Table 1.

**[Table 1]**

| Manufacturer | Name of antibody | Name of clone | Immunized animal |
|---|---|---|---|
| Calbiochem | Anti-5-Methylcytosine Mouse mAb | 162 33D3 | Mouse |
| abcam | 5-Methylcytidine | 33D3 | Mouse |
| Eurogentec | 5-Methylcytidine | monoclonal | Mouse |
| Aviva System Biology | 5-Methylcytosine | 33D3 | Mouse |
| Novus Biologicals | Cytosine (5-Methyl) | polyclonal | Sheep |
| Diagenode | 5-methyl cytidine | monoclonal | Mouse |

In the embodiments of the present invention, an anti-methylated DNA antibody produced by a known method in the art may be used. The anti-methylated DNA antibody can be produced, for example, by the following procedure. First, an appropriate mammal (e.g. rats and mice) is immunized with methylated DNA such as 5-methylcytosine as an immunogen together with an adjuvant, if desired. Then, an antibody-producing cell that produces an antibody against an immunogen is selected by screening it from spleen cells of the immunized animal. The obtained antibody-producing cell is fused with a myeloma cell to produce a hybridoma. The hybridoma is screened and thus a hybridoma that produces an antibody having a specific binding activity to methylated DNA is obtained. The anti-methylated DNA antibody can be obtained from the ascitic fluid obtained by administering the culture supernatant of the obtained hybridoma or the hybridoma to the abdominal cavity of a mouse.

Examples of the hybridoma for producing an anti-methylated DNA antibody include hybridoma SCR2 deposited with The National Institute of Technology and Evaluation (2-5-8 Kazusakamatari, Kisarazu-shi, Chiba 292-0818, Japan.) under Accession No. NITE BP-805 on August 25, 2009; and hybridomas SCR1, SCR3, and SCR6 deposited under Accession Nos. NITE BP-810, NITE BP-811, and NITE BP-812, respectively on September 10, 2009.
In the embodiments of the present invention, anti-methylated DNA antibodies which are produced from the hybridomas SCR1, SCR2, SCR3, and SCR6 can be used.

Examples of a methylated DNA binding protein include MBD1 (methyl cytosine binding domainprotein 1), MBD2 (methyl cytosine binding domain protein 2), MBD4 (methyl cytosine binding domain protein 4), and MeCP2(methyl CpG binding protein 2). These proteins in themselves are known in the art.
In the embodiments of the present invention, the methylated DNA binding protein may be a variant having deletion, substitution, or addition of one or more amino acids in an amino acid sequence of a wild type as long as it can specifically recognize and bind to methylated DNA. A method of producing such a variant itself is known in the art.

In the embodiments of the present invention, the contact of the sample which may include methylated DNA with the protein capable of binding to methylated DNA can be performed by adding the protein to a sample. In the embodiments of the present invention, the additive amount of the protein capable of binding to methylated DNA is not particularly limited and it may be an additive amount capable of ensuring the amount of methylated DNA which can be sufficiently detected in the following detection process. For example, when the content of nucleic acid in a sample is from 1 ng to 10 μg, the additive amount of the anti-methylated DNA antibody may be from about 1 ng to 10 μg.
The contact conditions (ambient temperature and time) vary depending on the type of the protein capable of binding to methylated DNA and are usually as follows: at 4 to 42°C for about 10 minutes to 24 hours.

In the detection method of the present invention, the sample obtained in the binding process is brought into contact with at least one deoxyribonuclease to degrade DNA in the sample (hereafter the process is called a "degradation process").
In the process, DNA which is not bound to the protein capable of binding to methylated DNA (i.e., unmethylated DNA) is degradated by deoxyribonuclease. On the other hand, in the case of methylated DNA bound to the protein, 5-methylcytosine and its peripheral region is not degradated by the binding.
That is, in the detection method of the present invention, unmethylated DNA can be removed by the binding process and the degradation process.

In the embodiments of the present invention, deoxyribonuclease may be either of endonuclease or exonuclease. In the embodiments of the present invention, a restriction enzyme may be used as deoxyribonuclease. In this case, a DNA cleavage site is limited to a recognition region of the restriction enzyme. Therefore, when the sample obtained by the degradation process is directly used for the subsequent detection process, it should be noted that degradated products have no affect on the following detection process. For example, unmethylated DNA can be sufficiently degradated using a plurality of restriction enzymes.

In the preferred embodiment of the present invention, deoxyribonuclease is at least one selected from the group consisting of deoxyribonuclease I (DNaseI), exonuclease I, lambda exonuclease, T7 exonuclease, exonuclease III, RecJ exonuclease, exonuclease T, BAL31 nuclease, Mung Bean nuclease, the Micrococcaceae nuclease, and T7 endonuclease.

In the detection method of the present invention, at least one deoxyribonuclease to be used is a deoxyribonuclease different from a methylation-sensitive restriction enzyme capable of degradating single-stranded DNA. The term "methylation-sensitive restriction enzyme capable of degradating a single-stranded DNA" means a restriction enzyme that does not cleave a recognition sequence containing methylated cytosine in a single-stranded DNA and can cleave only a recognition sequence containing unmethylated cytosine. As such a restriction enzyme, for example, HhaI is cited. In particular embodiments of the detection methods of the present invention at least one methylation insensitive deoxyribonuclease is used.

In the embodiments of the present invention, the contact of the sample obtained in the binding process with deoxyribonuclease can be performed by adding deoxyribonuclease to a sample. In the embodiments of the present invention, the additive amount of deoxyribonuclease is not particularly limited and it may be an additive amount capable of sufficiently degrading unmethylated DNA in a subsequent detection process. For example, when the content of nucleic acid in a sample is from 1 ng to 10 μg, the additive amount of DNaseI may be from about 0.1 to 120 U.

The contact conditions (ambient temperature and time) vary depending on the type of deoxyribonuclease and are usually as follows: at 4 to 42°C for about 10 minutes to 24 hours.

In the detection method of the present invention, the sample obtained by the degradation process can be directly used in the following detection process. Thus, the process of cleaning to remove unmethylated DNA and the process of purifying methylated DNA can be omitted. Thus in particular embodiments, the methods of the invention do not comprise a step of separating methylated from unmethylated DNA prior to detection of methylated DNA. In more particular embodiments of the methods of the invention the step of detecting methylated DNA which is not degraded by the deoxyribonuclease (due to the binding of the sample obtained by the degradation process to the protein) follows directly after the step of bringing the sample (obtained in the binding process) into contact with at least one deoxyribonuclease to degrade DNA in the sample.

In the embodiments of the present invention, depending on a detection means to be used in the following detection process, a nucleic acid amplification method may be executed using methylated DNA included in the sample obtained by the degradation process as a template. The DNA amplification method itself is not particularly limited as long as it is a known DNA amplification method in the art. Examples thereof include *in vitro* transcription (IVT) amplification, SPIA (trademark) amplification, and GenomiPhi amplification.

In the detection method of the present invention, methylated DNA which is not degraded by deoxyribonuclease is detected by the binding of the sample obtained by the degradation process with the protein capable of binding to methylated DNA (hereafter, the process is called as a "detection process").

The term "methylated DNA is detected" used herein means detection of the presence of methylation of a target CpG site in methylated DNA, determination of a base sequence of methylated DNA, and analysis of the frequency of methylation of the CpG site in methylated DNA. The term "frequency of methylation" used herein means the number or ratio of the methylated CpG site of all CpG sites or arbitrary CpG sites present in a target region in methylated DNA.

In the present specification, the detection of methylated DNA is not particularly limited as long as it is a method which can detect the methylated DNA which is not degraded by deoxyribonuclease in the degradation process, such as determination of methylation of a target DNA, determination of a base sequence of methylated DNA, and comprehensive analysis of methylated DNA.

In the embodiments of the present invention, the method to be used in the detection process is not particularly limited as long as it can detect the methylated DNA which is not degraded by deoxyribonuclease in the degradation process and it can be appropriately selected from known methods in the art. Examples of the method include a nucleic acid amplification method, a nucleotide sequencing method, and a microarray method.

For example, when the presence of methylation of a target DNA is determined by using the nucleic acid amplification method in the detection process, the PCR assay is performed using a primer set which is designed so that a CpG site in a target DNA is sandwiched and the presence of methylation of a target DNA is determined from the presence of amplified products. The content of methylated DNA in the sample can also be quantified by the quantitative PCR method. Further, if a high-speed sequencing method (a next-generation sequencing method), which is one of the nucleotide sequencing methods, is used in the detection process, methylated DNA can be comprehensively analyzed. Examples of a sequencer to be used for the high-speed sequencing method include ABI3730x1 (Life Technologies) and GS FLX Titanium (Roche).

The microarray method can also be used in the detection process. In this case, a microarray is not particularly limited and a DNA microarray or a DNA chip is preferred. A commercially available microarray such as GeneChip (registered trademark, manufactured by Affymetrix) or a microarray produced by a known method in the art may be used. If a tiling array which is a microarray in which probes having a base sequence extracted at equal intervals from a whole genome region or a specific region are disposed in a tile shape is used in the detection process, methylated DNA can be comprehensively analyzed.

In the embodiments of the present invention, when the microarray method is used in the detection process, the DNA in the sample is preferably labeled with a labeling substance known in the art. Therefore, the detection method of the present invention may further include a process of labeling DNA in a sample. Examples of the labeling substance include fluorescent substances, haptens such as biotin, and radioactive substances. Examples of the fluorescent substances include Cy3, Cy5, Alexa Fluor (trademark), and FITC. In the microarray method, the signal is easily measured by labeling DNA. Methods for labeling the DNA with the labeling substance are known in the art.

The signals may be signals suitable for the type of the microarray. For example, the signals may be electric signals which are generated in the presence of DNA hybridizing to each probe of the microarray, or when the DNA in the sample is labeled as described above, the signals may be fluorescence signals, luminescence signals or radioactive signals.
Each of the signals may be detected using a scanner installed in a general microarray analyzer. For example, the scanner may be GeneChip (registered trademark), Scanner 3000 7G (Affymetrix, Inc.) or the like.

Hereinafter, the present invention will be described in detail with reference to examples, however, the present invention is not limited to the examples.

### [Examples]

### (Example 1)

As a target methylated DNA, 6MeCG oligonucleotide, i.e., oligonucleotide containing six of 5-methylcytosine was used. The base sequence of 6MeCG oligonucleotide is shown below. ("m5c" in the base sequence represents 5-methylcytosine.))

### (1) Preparation of sample

A 10 pM concentration of an aqueous solution of 6MeCG oligonucleotide was prepared as a 6MeCG oligonucleotide solution. The 6MeCG oligonucleotide solution was heated at 95°C for 10 minutes and the modified solution was allowed to stand on ice for 1 minute. 2 μl was taken from the modified 6MeCG oligonucleotide solution and this was used as an input sample. The remaining solution was used as a sample to be subjected to the detection method of the present invention.

### (2) Contact of sample with anti-methylated DNA antibody

The sample was divided into two. An anti-methylated DNA antibody (1 μg) was added to one of them to prepare a specimen sample (100 μl) and the anti-methylated DNA antibody was not added to the other one to prepare a control sample (100 μl). Each sample was incubated at 25 °C for 2 hours.

The anti-methylated DNA antibody used in this example is a monoclonal antibody obtained from hybridoma SCR2 deposited with The National Institute of Technology and Evaluation (2-5-8 Kazusakamatari, Kisarazu-shi, Chiba 292-0818, Japan.) under Accession No. NITE BP-805 on August 25, 2009.

### (3) Degradation of DNA by deoxyribonuclease

1 μl of DNaseI, i.e., a type of deoxyribonuclease (2 U/μl:NEB) was added to the specimen sample and the control sample and each mixture was reacted at 37°C for 1 hour.
After the reaction, each sample was heated at 75°C for 10 minutes to deactivate DNaseI.

### (4) Detection of methylated DNA

In order to examine whether 6MeCG oligonucleotide remained in a sample, the quantitative PCR method was performed.

### (i) Preparation of PCR reaction solution

The following reagents were mixed to prepare 12 μl of a reaction solution.

| | |
|---|---|
| 2x FastStart SYBR Green Master Mix (ROCHE) | 6 µl |
| Forward (F) primer (10 µM) | 1 µl |
| Reverse (R) primer (10 µM) | 1 µl |
| Sample | 1 µl |
| dH₂O | 3 µl |
| Total | 12 µl |

### (ii) Sequences of primers

As a primer set for detecting 6MeCG oligonucleotide, a primer set represented by SEQ ID NO: 2 and SEQ ID NO: 3 was used. The base sequences of the primer set are shown below.
F:5'-CGAGGTCGACGGTAT-3' (SEQ ID NO: 2)
R:5'-CCAGTCACGACGTTGTA-3' (SEQ ID NO: 3)

### (iii) Quantitative PCR reaction conditions

The above reaction solution was subjected to quantitative PCR under the following reaction conditions using Mx3005P (manufactured by Stratagene).
45 cycles of PCR at 95°C for 10 minutes, at 95°C for 30 seconds, and at 55 °C for 15 seconds, at 72°C for 30 seconds; and 1 cycle of PCR at 95°C for 1 minute, at 55°C for 30 seconds, at 95°C for 30 seconds.

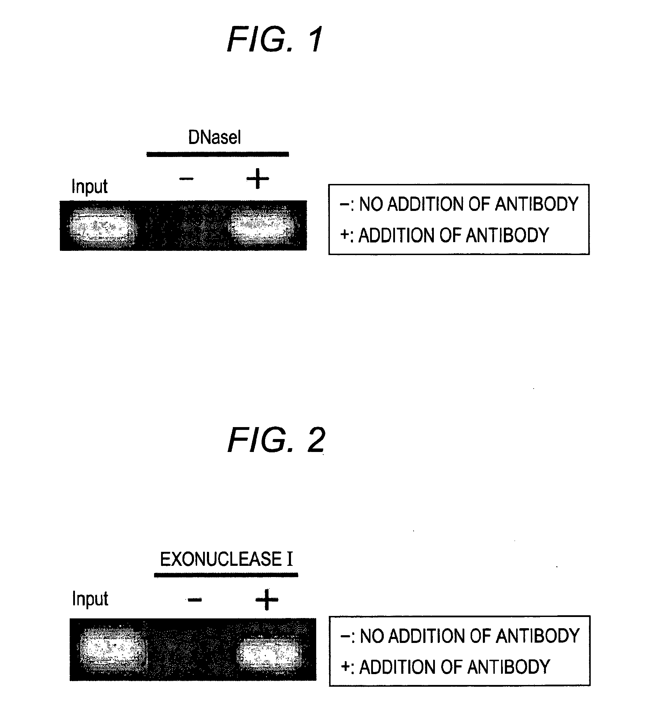

The obtained reaction solution was subjected to electrophoresis using 3% agarose gel and then the presence of amplified products was confirmed. The results are shown in FIG. 1.
From Fig. 1, it is found that bands of amplified products were not detected in the sample to which the anti-methylated DNA antibody was not added, while the bands were detected in the sample to which the antibody was added. This suggested that the degradation of the oligonucleotide by DNaseI was inhibited by allowing the anti-methylated DNA antibody to be bound to 6MeCG oligonucleotide, and thus 6MeCG oligonucleotide could be detected.

### (Example 2)

### (1) Preparation of sample

A solution of 6MeCG oligonucleotide was prepared in the same manner as described in Example 1, and an input sample and a sample being subjected to the detection method of the present invention were obtained from the solution.

### (2) Contact with anti-methylated DNA antibody

Specimen and control samples were obtained from the above sample in the same manner as described in Example 1.

### (3) Degradation of DNA by deoxyribonuclease

4 μl of Exonuclease I (20 U/μl:NEB), i.e., a type of deoxyribonuclease was added to each of the specimen and control samples and each mixture was reacted at 37°C for 1 hour. After the reaction, each sample was heated at 80°C for 20 minutes to deactivate Exonuclease I.

### (4) Detection of methylated DNA

In order to examine whether 6MeCG oligonucleotide remained in a sample, the quantitative PCR method was performed in the same manner as described in Example 1.

The obtained reaction solution was subjected to electrophoresis using 3% agarose gel and then the presence of amplified products was confirmed. The results are shown in Fig. 2.

From Fig. 2, it is found that bands of amplified products were not detected in the sample to which the anti-methylated DNA antibody was not added, while the bands were detected in the sample to which the antibody was added. This suggested that the degradation of the oligonucleotide by Exonuclease I was inhibited by allowing the anti-methylated DNA antibody to be bound to 6MeCG oligonucleotide, and thus 6MeCG oligonucleotide could be detected.

### (Example 3)

In this example, as the target methylated DNA, a promoter region of GSTP1 gene which was known to be frequently modified by methylation in genomic DNA of the breast cancer cell line MCF7 was selected. The base sequence in the promoter region of GSTP1 gene is known in the art.

As the unmethylated DNA (negative control), a region which was present in the 14th human chromosome and was not modified by methylation because of having no CpG site (hereinafter referred to as "CGF-1 region") was selected. The base sequence of the CGF-1 region is shown below.

### <CGF-1 region>

### (1) Preparation of sample

A solution of genomic DNA extracted from MCF7 cells was fragmented by ultrasonic fragmentation using Bio-raptor (registered trademark, manufactured by COSMO BIO Co., Ltd.) to prepare a solution of genomic DNA fragments with 500 bp or less. The prepared solution of genomic DNA fragments was modified by heating at 95°C for 10 minutes and the resultant solution was allowed to stand on ice for 1 minute to obtain a solution of single stranded DNA fragments.

An aliquot was taken from a sample prepared by adjusting the obtained solution of single stranded DNA fragments so that the amount of DNA was 2 ng/μl and the aliquot was used as an input sample.

### (2) Contact of sample with anti-methylated DNA antibody

The anti-methylated DNA antibody (1 μg) obtained from hybridoma SCR2 was added to50 μl of the sample and the mixture was used as a specimen sample (100 μl). Pure water was added to 50 μl of the sample and the mixture was used as a control sample (100 μl). Each sample was incubated at 25 °C for 2 hours.

### (3) Degradation of DNA by deoxyribonuclease

The specimen sample was divided into two. 0.5 μl of DNaseI (2 U/μl:NEB) was added one of them and 1 μl of DNaseI was added to the other one. Similarly, the control sample was divided into two. 0.5 μl of DNaseI (2 U/μl:NEB) was added one of them and 1 μl of DNaseI was added to the other one. These samples were reacted at 37°C for 1 hour. After the reaction, each sample was heated at 75°C for 10 minutes to deactivate DNaseI.

### (4) Detection of methylated DNA

In order to examine whether methylated DNA derived from a genome of MCF7 in a sample remained, the quantitative PCR method was performed. In order to confirm that methylated DNA is specifically detected by the detection method of the present invention, degradation of unmethylated DNA in a sample was examined by the quantitative PCR method.

### (i) Preparation of PCR reaction solution

The following reagents were mixed to prepare 12 μl of a reaction solution.

| | |
|---|---|
| 2x FastStart SYBR Green Master Mix (ROCHE) | 6 µl |
| Forward (F) primer (10 µM) | 1 µl |
| Reverse (R) primer (10 µM) | 1 µl |
| Sample | 1 µl |
| dH₂O | 3 µl |
| Total | 12 µl |

### (ii) Sequences of primers

As a primer set for detecting methylated DNA, a primer set for amplifying a promoter region of GSTP1 gene was used. The base sequences of the primer set are shown below.
F:5'- GAGGCCTTCGCTGGAGTT -3'(SEQ ID NO 5)
R:5'- GTACTCACTGGTGGCGAAGA -3'(SEQ ID NO 6)

As a primer set for detecting unmethylated DNA, a primer set for amplifying a CGF-1 region was used. The base sequences of the primer set are shown below.
F:5'- GGAGGAGTCAAGAGAAGTTGGAAGC -3'(SEQ ID NO 7)
R:5'- CCCACACTCCATTTCCATTCCTC -3'(SEQ ID NO 8)

### (iii) Quantitative PCR reaction conditions

The above reaction solution was subjected to quantitative PCR under the following reaction conditions using Mx3005P (manufactured by Stratagene).
45 cycles of PCR at 95°C for 10 minutes, at 95°C for 30 seconds, and at 66 °C for 15 seconds, at 72°C for 30 seconds; and 1 cycle of PCR at 95°C for 1 minute, at 66°C for 30 seconds, at 95°C for 30 seconds.

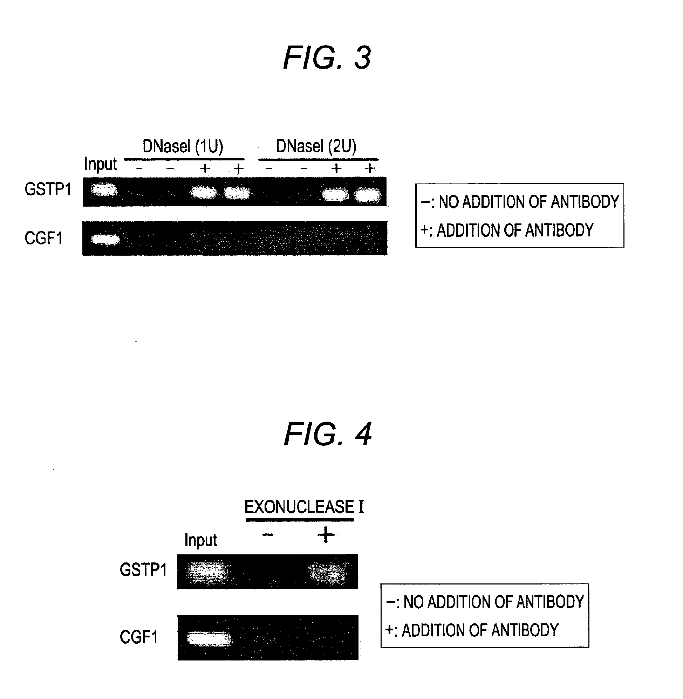

The obtained reaction solution was subjected to electrophoresis using 3% agarose gel and then the presence of amplified products was confirmed. The results are shown in Fig. 3.
From Fig. 3, it is found that bands of amplified products in the GSTP1 promoter region were not detected in the sample to which the anti-methylated DNA antibody was not added, while the bands were detected in the sample to which the antibody was added. On the other hand, it is found that bands of amplified products in the CGF-1 region were not detected regardless of whether the addition of the anti-methylated DNA antibody was carried out.
This shows that the degradation of methylated DNA by DNaseI is inhibited by allowing the anti-methylated DNA antibody to be bound to a region where the genomic DNA is methylated, and thus methylated DNA can be detected. On the other hand, it is suggested that the phenomenon in which the degradation reaction by DNaseI caused by binding to the anti-methylated DNA antibody is inhibited specifically occurs in the region to be methylated.

### (Example 4)

### (1) Preparation of sample

A solution containing a single-stranded genomic DNA derived from MCF7 was prepared in the same manner as described in Example 3 and an input sample and a sample being subjected to the detection method of the present invention were obtained from the solution.

### (2) Contact with anti-methylated DNA antibody

Specimen and control samples were obtained from the above sample in the same manner as described in Example 3.

### (3) Degradation of DNA by deoxyribonuclease

2.5 μl of Exonuclease I (20 U/μl:NEB) was added to each of the specimen and control samples, followed by reaction at 37°C for 1 hour. After the reaction, each sample was heated at 80°C for 20 minutes to deactivate Exonuclease I.

### (4) Detection of methylated DNA

In order to examine whether methylated DNA derived from a genome of MCF7 in a sample remained, a PCR reaction solution was prepared in the same manner as described in Example 3 and the quantitative PCR method was performed. The reaction conditions of quantitative PCR are as follows.

### <Quantitative PCR using primer set for amplifying promoter region of GSTP1>

35 cycles of PCR at 95°C for 10 minute, at 95°C for 30 seconds, at 66°C for 15 seconds, at 72°C for 30 seconds; and 1 cycle of PCR at 95°C for 1 minute, at 66°C for 30 seconds, at 95°C for 30 seconds.

### <Quantitative PCR using primer set for amplifying CGF-1 region>

45 cycles of PCR at 95°C for 10 minutes, at 95°C for 30 seconds, and at 66 °C for 15 seconds, at 72°C for 30 seconds; and 1 cycle of PCR at 95°C for 1 minute, at 66°C for 30 seconds, at 95°C for 30 seconds.

The obtained reaction solution was subjected to electrophoresis using 3% agarose gel and then the presence of amplified products was confirmed. The results are shown in Fig. 4.
From Fig. 4, it is found that bands of amplified products in the GSTP1 promoter region were not detected in the sample to which the anti-methylated DNA antibody was not added, while the bands were detected in the sample to which the antibody was added. On the other hand, it is found that bands of amplified products in the CGF-1 region were not detected regardless of whether the addition of the anti-methylated DNA antibody was carried out.
This shows that the degradation of methylated DNA by Exonuclease I is inhibited by allowing the anti-methylated DNA antibody to be bound to a region where the genomic DNA is methylated, and thus methylated DNA can be detected. On the other hand, it is suggested that the phenomenon in which the degradation reaction by Exonuclease I caused by binding to the anti-methylated DNA antibody is inhibited specifically occurs in the region to be methylated.

## Claims

1. A method of detecting methylated DNA in a sample, comprising:
bringing a sample which may include methylated DNA into contact with a protein capable of binding to methylated DNA to bind the methylated DNA in the sample to the protein;
bringing the sample obtained in the binding process into contact with at least one deoxyribonuclease to degrade DNA in the sample; and
detecting methylated DNA which is not degraded by the deoxyribonuclease by the binding of the sample obtained by the degradation process to the protein ; wherein
the at least one deoxyribonuclease is a deoxyribonuclease different from a methylation-sensitive restriction enzyme capable of degradating single-stranded DNA.

2. The method according to claim 1, wherein
a protein capable of binding to the methylated DNA is an anti-methylated DNA antibody or a methylated DNA binding protein.

3. The method according to claim 1, wherein
the protein capable of binding to the methylated DNA is an anti-methylated DNA antibody.

4. The method according to claim 1, wherein
the protein capable of binding to the methylated DNA is at least one methylated DNA binding protein selected from the group consisting of MBD1, MBD2, MBD4, and MeCP2.

5. The method according to any one of claims 1 to 4, wherein
the sample is cultured cells or a sample prepared from blood, body fluids, tissues or cells collected from organisms.

6. The method according to any one of claims 1 to 5, wherein
the deoxyribonuclease is at least one selected from the group consisting of deoxyribonuclease I, exonuclease I, lambda exonuclease, T7 exonuclease, exonuclease III, RecJ exonuclease, exonuclease T, BAL31 nuclease, Mung Bean nuclease, the Micrococcaceae nuclease, and T7 endonuclease.

7. The method according to any one of claims 1 to 6, wherein
the sample is a sample which may include single-stranded methylated DNA and
single-stranded methylated DNA is detected in the process of detecting methylated DNA.

8. The method according to any one of claims 1 to 7, wherein
methylated DNA is detected using a nucleic acid amplification method, a nucleotide sequencing method or a microarray method in the process of detecting methylated DNA.

## Patentansprüche

1. Verfahren zum Nachweis methylierter DNA in einer Probe, welches Folgendes umfasst:
in Kontakt bringen einer Probe, welche möglicherweise methylierte DNA enthält, mit einem Protein, welches in der Lage ist, an methylierte DNA zu binden, zum Binden der methylierten DNA in der Probe an das Protein;
in Kontakt bringen der Probe, welche in dem Bindungsprozess erhalten wurde, mit mindestens einer Deoxyribonuklease zum Zersetzen von DNA in der Probe; und
Nachweisen methylierter DNA, welche nicht durch die Deoxyribonuklease zersetzt wird, durch die Bindung der Probe, welche durch den Zersetzungsprozess erhalten wurde, an das Protein;
wobei die mindestens eine Deoxyribonuklease eine andere Deoxyribonuklease als ein methylierungssensitives Restriktionsenzym, welches in der Lage ist, Einzelstrang-DNA zu zersetzen, ist.

2. Verfahren nach Anspruch 1, wobei ein Protein, welches zum Binden an die methylierte DNA in der Lage ist, ein Antikörper gegen methylierte DNA oder ein Bindungsprotein für methylierte DNA ist.

3. Verfahren nach Anspruch 1, wobei das Protein, welches zum Binden an die methylierte DNA in der Lage ist, ein Antikörper gegen methylierte DNA ist.

4. Verfahren nach Anspruch 1, wobei das Protein, welches zum Binden an die methylierte DNA in der Lage ist, mindestens ein Bindungsprotein für methylierte DNA ausgewählt aus der Gruppe bestehend aus MBD1, MBD2, MBD4 und MeCP2 ist.

5. Verfahren nach einem der Ansprüche 1 bis 4, wobei die Probe kultivierte Zellen oder eine Probe hergestellt aus Blut, Körperfluiden, Geweben oder Zellen entnommen aus Organismen sind/ist.

6. Verfahren nach einem der Ansprüche 1 bis 5, wobei die Deoxyribonuklease mindestens eine ausgewählt aus der Gruppe bestehend aus Deoxyribonuklease I, Exonuklease I, Lambda-Exonuklease, T7-Exonuklease, Exonuklease III, RecJ-Exonuklease, Exonuklease T, BAL31-Nuklease, Mungobohnen-Nuklease, der Mikrokokken-Nuklease und T7-Endonuklease ist.

7. Verfahren nach einem der Ansprüche 1 bis 6, wobei die Probe eine Probe ist, welche methylierte Einzelstrang-DNA enthalten kann, und die methylierte Einzelstrang-DNA in dem Prozess des Nachweises methylierter DNA nachgewiesen wird.

8. Verfahren nach einem der Ansprüche 1 bis 7, wobei methylierte DNA mittels eines Nukleinsäure-Amplifikationsverfahrens, eines Nukleotidsequenzierungsverfahrens oder eines Mikroarrayverfahrens im Prozess des Nachweises methylierter DNA nachgewiesen wird.

## Revendications

1. Procédé de détection d'ADN méthylé dans un échantillon, ledit procédé comprenant :
la mise en contact d'un échantillon qui peut contenir de l'ADN méthylé avec une protéine capable de se lier à l'ADN méthylé pour lier l'ADN méthylé dans l'échantillon à la protéine ;
la mise en contact de l'échantillon obtenu dans le processus de liaison avec au moins une désoxyribonucléase pour dégrader l'ADN dans l'échantillon ; et
la détection de l'ADN méthylé qui n'est pas dégradé par la désoxyribonucléase par la liaison à la protéine de l'échantillon obtenu par le processus de dégradation ; dans lequel
la ou les désoxyribonucléases sont une désoxyribonucléase autre qu'une enzyme de restriction sensible à la méthylation capable de dégrader l'ADN monocaténaire.

2. Procédé selon la revendication 1, dans lequel une protéine capable de se lier à l'ADN méthylé est un anticorps anti-ADN méthylé ou une protéine liant l'ADN méthylé.

3. Procédé selon la revendication 1, dans lequel la protéine capable de se lier à l'ADN méthylé est un anticorps anti-ADN méthylé.

4. Procédé selon la revendication 1, dans lequel la protéine capable de se lier à l'ADN méthylé est au moins une protéine liant l'ADN méthylé choisie dans le groupe consistant en MBD1, MBD2, MBD4 et MeCP2.

5. Procédé selon l'une quelconque des revendications 1 à 4, dans lequel l'échantillon est des cellules cultivées ou un échantillon préparé à partir de sang, de fluides biologiques, de tissus ou de cellules prélevés sur des organismes.

6. Procédé selon l'une quelconque des revendications 1 à 5, dans lequel la désoxyribonucléase est au moins une choisie dans le groupe consistant en la désoxyribonucléase I, l'exonucléase I, l'exonucléase lambda, l'exonucléase T7, l'exonucléase III, l'exonucléase RecJ, l'exonucléase T, la nucléase BAL31, la nucléase de haricot mungo, la nucléase de Micrococcaceae, et l'endonucléase T7.

7. Procédé selon l'une quelconque des revendications 1 à 6, dans lequel l'échantillon est un échantillon qui peut comprendre de l'ADN simple brin méthylé et l'ADN simple brin méthylé est détecté durant le processus de détection d'ADN méthylé.

8. Procédé selon l'une quelconque des revendications 1 à 7, dans lequel l'ADN méthylé est détecté en utilisant un procédé d'amplification d'acide nucléique, un procédé de séquençage de nucléotides ou un procédé sur micropuce dans le processus de détection d'ADN méthylé.


### SEQUENCE LISTING

<110> Sysmex Corporation
<120> Method for detecting methylated DNA in sample
<130> SYSM-062-EP
<160> 8
<170> PatentIn version 3.5
<210> 1
   <211> 73
   <212> DNA
   <213> Artificial Sequence
<220>
   <223> 6MeCG oligonucleotide
<220>
   <221> modified_base
   <222> (20)..(20)
   <223> m5c
<220>
   <221> modified_base
   <222> (27)..(27)
   <223> m5c
<220>
   <221> modified_base
   <222> (34)..(34)
   <223> m5c
<220>
   <221> modified_base
   <222> (40)..(40)
   <223> m5c
<220>
   <221> modified_base
   <222> (46)..(46)
   <223> m5c
<220>
   <221> modified_base
   <222> (52).. (52)
   <223> m5c
<400> 1
   cgaggtcgac ggtattgatc gagtatcgat agtcgatatc gatatcgata tcgatataca 60
   acgtcgtgac tgg 73
<210> 2
   <211> 15
   <212> DNA
   <213> Artificial Sequence
<220>
   <223> primer
<400> 2
   cgaggtcgac ggtat 15
<210> 3
   <211> 17
   <212> DNA
   <213> Artificial Sequence
<220>
   <223> primer
<400> 3
   ccagtcacga cgttgta 17
<210> 4
   <211> 193
   <212> DNA
   <213> Homo sapiens
<400> 4
   ggaggagtca agagaagttg gaagccaact gagagagagg gaaggcttga agtggtcagg      60
   acagtgaaca cctaagagac atccactgaa tttgcccact aggaagccat tagtgacttc     120
   aataggaaca tcttcagtgc atcatgaagg ccaaagattg ccatgaaaga gaggaatgga     180
   aatggagtgt ggg                                                         193
<210> 5
   <211> 18
   <212> DNA
   <213> Artificial Sequence
<220>
   <223> primer
<400> 5
   gaggccttcg ctggagtt 18
<210> 6
   <211> 20
   <212> DNA
   <213> Artificial Sequence
<220>
   <223> primer
<400> 6
   gtactcactg gtggcgaaga 20
<210> 7
   <211> 25
   <212> DNA
   <213> Artificial Sequence
<220>
   <223> primer
<400> 7
   ggaggagtca agagaagttg gaagc 25
<210> 8
   <211> 23
   <212> DNA
   <213> Artificial Sequence
<220>
   <223> primer
<400> 8
   cccacactcc atttccattc ctc 23